# EUROPÄISCHE PATENTSCHRIFT

(11) **EP 4 376 758 B1**
(45) Veröffentlichungstag und Bekanntmachung des Hinweises auf die Patenterteilung: **05.03.2025**
(21) Anmeldenummer: 22758151.9
(22) Anmeldetag: 25.07.2022
(51) Int. Cl.: A61B 34/30, A61B 34/00, A61B 1/005, A61B 17/00, A61B 17/29

(54) **CHIRURGISCHES INSTRUMENT UND LENKGETRIEBE DAFÜR**
SURGICAL INSTRUMENT AND STEERING MECHANISM THEREFOR
INSTRUMENT CHIRURGICAL ET MÉCANISME DE DIRECTION S'Y RAPPORTANT

(30) Priorität: 28.07.2021 DE 102021119527
(43) Veröffentlichungstag der Anmeldung: 05.06.2024
(73) Patentinhaber: Karl Storz SE & Co. KG, 78532 Tuttlingen (DE)
(72) Erfinder: SCHNEIDER, Janosz, 78532 Tuttlingen (DE); LÄNGLE, Dominik, 78532 Tuttlingen (DE); STEFAN, Jochen, 78532 Tuttlingen (DE)
(86) Internationale Anmeldenummer: PCT/EP2022/070791
(87) Internationale Veröffentlichungsnummer: WO 2023/006664

(56) Entgegenhaltungen:
- WO-A2-2020/218920
- DE-A1- 102019 121 092
- US-A- 5 454 827
- US-A1- 2005 090 809

## Beschreibung

Die Erfindung betrifft ein chirurgisches Instrument und ein Lenkgetriebe dafür.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinkelungsrichtungen erzielt werden.

Ein gattungsgemäßes chirurgisches Instrument ist bspw. aus der US 5 454 827 bekannt, bei dem die distalseitigen Schwenkglieder über vier Lenkdrähte mit einer proximalseitig angeordneten räumlich verstellbaren Taumelscheibe so gekoppelt sind, dass eine Bewegung der räumlich verstellbaren Taumelscheibe eine entsprechende relative Bewegung der distalseitigen Schwenkglieder und somit ein Verschwenken der Werkzeugspitze verursacht wird, wobei das Bewegen der räumlich verstellbaren Taumelscheibe manuell über eine Art Joystick erfolgt, der direkt mit dieser gekoppelt ist.

Die Ausbildung des Antriebs für die Lenkdrähte mit der räumlich verstellbaren Taumelscheibe, an der alle Lenkdrähte gelagert sind, hat den Vorteil, dass dies eine räumlich kompakte Bauweise ermöglicht und nur ein Bauteil bewegt werden muss, um alle Lenkdrähte ansprechen zu können.

In der US 7 699 855 ist ein chirurgisches Instrument offenbart, das eine Schnittstelle aufweist, um das Instrument mit einem Roboter-Arm verbinden zu können. Dabei sind alle Antriebe, die das Instrument steuern, in dem Roboter-Arm angeordnet. Die Übertragung der Drehwinkel von Antrieben zum Instrument erfolgt über Kupplungsscheiben in einer gemeinsamen Trenn-Ebene.

Die WO 2014/004242 beschreibt ebenfalls eine solche Schnittstelle, wobei die Antriebe in dem Roboter-Arm verbaut sind.

Die vorstehende Gestaltung ist verbunden mit einem komplexen Aufbau und einer indirekten und spielbehafteten Ansteuerung. Die Antriebe sind nicht in dem chirurgischen Instrument direkt angeordnet, woraus ein nicht lineares Übertragungsverhalten bei der Ansteuerung der Taumelscheibe resultiert, das nur schlecht in einer Software abgebildet werden kann.

Die US 10,105,128 B2 offenbart auch eine Ansteuerung einer solchen Werkzeugspitze; dort erfolgt dies über eine Mechanik, die Zahnscheiben-Segmente und Gelenkstangen umfasst, um die Bewegung der Antriebe auf die Taumelscheibe zu übertragen.

Die DE 10 2019 121 092 A1 beschreibt ein medizinisches Instrument mit einem hohlen Schaft, an dessen distalen Ende ein angeordnetes Instrument mittels eines Gelenkmechanismus relativ zur Längsachse des Schaftes schwenkbar ist, wobei der Gelenkmechanismus über in Längsrichtung des Schafts verlaufende Lenkdrähte mit einem proximalen Antrieb verbunden ist, wobei die Lenkdrähte an einer räumlich verstellbaren Scheibe des proximalen Antriebs gelagert sind und der motorisierte Antrieb aus zwei um 180° zueinander versetzt angeordneten Antriebseinheiten besteht, deren Antriebswellen auf einer gemeinsamen Mittelachse liegen, und die räumlich verstellbare Scheibe zwischen den zwei Antriebseinheiten angeordnet ist. Die Antriebseinheiten sind jeweils als über Motoren angetriebene Zahnräder ausgebildet, wobei jedes der beiden Zahnräder als einfaches Antriebsrad mit nur einem Zahnradkranz ausgebildet ist. Bevorzugt greifen die beiden angetriebenen Zahnräder der Antriebseinheiten in ein drittes Zahnrad ein, welches mit der räumlich verstellbaren Scheibe gekoppelt ist.

Das Dokument US 2005/090809 A1 betrifft unterschiedliche Ausführungsformen von proximalseitigen und distalseitigen Gelenkmechaniken eines medizinischen Instrumentes. Im Falle eines kardanisch aufgehängten Kabelaktuators mit in Pitch-Rotation konfigurierten Aktuatorgliedern ist die Betätigungsplatte, an welcher Lenkkabel verbunden sind, derart kardanisch mittels einer Halterung aufgehängt, dass eine Neigung der Betätigungsplatte in zwei Freiheitsgraden möglich ist. Die Betätigungsplatte ist mit zwei angetriebenen Betätigungsgliedern verbunden, wobei jedes Betätigungsglied über Drehgelenke mit einem drehbaren Zahnradquadranten verbunden ist, wobei jeder Zahnradquadrant durch jeweils ein Antriebszahnrad gedreht wird, welches wiederum durch eine Antriebsspule betätigt wird.

Ausgehend von diesem Stand der Technik ist es Aufgabe der vorliegenden Erfindung, ein verbessertes Lenkgetriebe für ein chirurgisches Instrument bereitzustellen, das einen Antrieb der räumlich verstellbare Taumelscheibe mit linearem Übertragungsverhalten hat und dabei platzsparend aufgebaut ist.

Diese Aufgabe wird durch ein Lenkgetriebe mit den Merkmalen des Anspruchs 1 gelöst.

Die weitere Aufgabe, ein chirurgisches Instrument bereitzustellen, dessen räumlich verstellbare Taumelscheibe durch ein konstruktiv einfaches und platzsparendes Lenkgetriebe angetrieben wird, wird durch das chirurgische Instrument mit den Merkmalen des unabhängigen Anspruchs 9 gelöst.

Weiterbildungen und bevorzugte Ausführungsformen des Lenkgetriebes und des chirurgischen Instruments sind in den Unteransprüchen ausgeführt.

Gemäß einer ersten Ausführungsform des erfindungsgemäßen Lenkgetriebes für ein chirurgisches Instrument ist das Lenkgetriebe am proximalen Ende eines Schafts angeordnet. Der Schaft definiert eine Längsachse B und weist am distalen Ende eine Abwinkelungsmechanik auf. Ferner weist das Lenkgetriebe zwei motorisierte Antriebe auf. Es ist dazu ausgebildet, über die Stellwinkel der zwei Antriebe eine Taumelscheibe räumlich auszurichten, die dazu ausgestaltet ist, die distale Abwinkelungsmechanik des chirurgischen Instruments zu steuern.

Erfindungsgemäß weist der erste Antrieb eine von einem ersten Motor angetriebene erste Antriebswelle auf, die mit einer ersten Zugmittelscheibe eines ersten Antriebsrads über ein erstes Zugmittel in Wirkverbindung steht. Das erste Zugmittel ist zwischen der ersten Zugmittelscheibe und einer Spindel angeordnet, die auf der ersten Antriebswelle sitzt. Die erste Antriebswelle definiert eine erste Antriebsachse C.

Der zweite Antrieb weist eine von einem zweiten Motor angetriebene zweite Antriebswelle auf, die mit einer zweiten Zugmittelscheibe eines zweiten Antriebsrads über ein zweites Zugmittel in Wirkverbindung steht. Das zweite Zugmittel ist zwischen der zweiten Zugmittelscheibe und einer Spindel angeordnet, die auf der zweiten Antriebswelle sitzt. Die zweite Antriebswelle definiert eine zweite Antriebsachse C'.

Dabei sind das erste und das zweite Antriebsrad als Doppelräder ausgebildet und weisen jeweils die entsprechende Zugmittelscheibe und einen an der jeweiligen rückwärtigen Seite vorliegenden Kegelradkranz auf. Zwischen den zwei Antriebsrädern, die eine gemeinsame Drehachse A haben, ist die Taumelscheibe angeordnet, wobei die Kegelradkränze einander zugewandt auf der Drehachse A angeordnet sind.

Durch die erfindungsgemäß um 90 ° in Bezug zu der gemeinsamen Achse der Doppelräder versetzte Anordnung der Motoren wird hinsichtlich der Ausrichtung der Taumelscheibe horizontaler Bauraum in Richtung der gemeinsamen Achse A der Doppelräder eingespart.

Vorteilhaft sind die Antriebsräder mit einer Doppelfunktionalität ausgestattet, so dienen sie nicht nur dazu die Taumelscheibe anzusteuern, sondern gleichzeitig als Bewegungsübertragungsmedien der Rotationsbewegung der Motoren auf weitere Bauteile des Lenkgetriebes und schließlich der Taumelscheibe. Damit wird eine direkte Umlenkung der Antriebsachsen erreicht, ohne eine zusätzliche Getriebestufe oder Spiel benötigt wird, wodurch eine kompakte Bauweise erzielt wird.

Bevorzugt sind Zugmittelscheibe und Kegelradkranz Rücken an Rücken zueinander angeordnet, wobei die Doppelräder besonders bevorzugt einstückig ausgebildet sind, wodurch sich eine kompakte und platzsparende Bauweise erreichen lässt.

Eine weitere Ausführungsform des erfindungsgemäßen Lenkgetriebes sieht vor, dass das erste Zugmittel und das zweite Zugmittel geschlossene oder nicht geschlossene Riemen oder Seile sind. So können Zugmittel mit unterschiedlichen Querschnitten verwendet werden, z. B. Seile mit rundem Querschnitt oder Riemen mit einem flachen, rechteckigen Querschnitt. Mehrgliedrige Ketten können ebenfalls geeignete Zugmittel sein. Die verwendeten Materialien sind abgestimmt auf die in dem Zugmittelantrieb aufzubringende Kraft; neben Zugmitteln aus zugfesten Metallen bzw. Metalllegierungen wie beispielsweise Stahlseilen, können auch Kunststoffe oder Verbundstoffe, wie Karbon oder andere faserverstärkte Kunststoffe genutzt werden. Die Form der Zugmittel kann eine geschlossene Form haben, so dass ein umlaufender Ring vorliegt. Nicht geschlossene Zugmittel können ebenfalls eingesetzt werden, wobei eine Befestigung der freien Enden sowohl an der Spindel als auch an der Zugmittelscheibe möglich ist. Die Zugmittelantriebe bieten eine zuverlässige Möglichkeit, die Taumelscheibe effektiv anzutreiben.

In einer bevorzugten Ausführungsform des erfindungsgemäßen Lenkgetriebes können die zwei Antriebsachsen C, C' parallel zueinander verlaufen, wobei die Antriebsachsen senkrecht zu der gemeinsamen Achse A verlaufen. Eine sogenannte achsparallele Anordnung der Motoren ermöglicht eine kompakte und damit platzsparende Anordnung der Komponenten des Lenkgetriebes. Ferner wird durch die parallele Anordnung der Motoren eine Anordnung nah an der Hauptachse des chirurgischen Instruments erreicht und so die Kraftübertragung verbessert. Eine alternative Ausführungsform des erfindungsgemäßen Lenkgetriebes sieht vor, dass jeder der Motoren über seine jeweilige Spindel in beliebiger Position radial von dem jeweiligen Antriebsradkranz wegweisend angeordnet werden kann. Eine beliebige Anordnung der Antriebe um die gemeinsame Drehachse der Doppelräder im Zusammenspiel mit einer geeigneten Wirkverbindung von Spindel und zugmittelscheibe erlaubt eine Vielzahl unterschiedlicher Anordnungen.

In noch einer weiteren Ausführungsform des erfindungsgemäßen Lenkgetriebes hat die Spindel eine umlaufende Nut, in der das Zugmittel geführt ist. Das Zugmittel liegt dabei in der Nut in einer gewissen Vorspannung, die sich durch das entsprechend gewählte Zugmittel und die Anordnung von Spindel und Zugmittelscheibe ergibt. Vorteilhaft wird so die Bewegung des Zugmittels geführt, so dass das Zugmittel nicht von der Spindel abrutschen kann.

Ferner sieht eine bevorzugte Ausführungsform des erfindungsgemäßen Lenkgetriebes vor, dass die Spindel eine Spindelmutter aufweist. Die Spindelmutter ist ein Sicherheitsbauteil, das wie die Nut ebenfalls verhindert, dass insbesondere bei schnellen Richtungswechseln der Bewegung des Zugmittels dieses nicht aus der Nut rutscht und ferner von der Spindel fällt.

Die Zugmittelscheibe weist in eine einer weiteren Ausführungsform des erfindungsgemäßen Lenkgetriebes eine umlaufende Nut auf, in der das Zugmittel anliegt. Wie schon für die Spindel, dient diese Nut zur gesicherten und gerichteten Führung des Zugmittels nun an der Zugmittelscheibe. Hiermit wird auch ermöglicht, dass durch Anordnung der Spindel in einem vorbestimmten Abstand das Zugmittel unter Zug gesetzt werden kann, so dass eine bestimmte Reibungskraft auf das Zugmittel ausgeübt wird. Das Zugmittel kann dadurch gezielter bewegt werden.

In noch einer weiteren oder auch alternativen Ausführungsform des erfindungsgemäßen Lenkgetriebes hat die Zugmittelscheibe eine umlaufende Nut aufweist, in der die Enden des nicht geschlossenen Zugmittels mit Nutensteinen befestigt sind. Diese Ausgestaltung ermöglicht die Verwendung von nicht geschlossenen Zugmitteln, deren freie Enden in der Nut der Zugmittelscheibe mit den Nutensteinen verliersicher befestigt sind.

In einer weiteren Ausführungsform des erfindungsgemäßen Lenkgetriebes kann die Taumelscheibe mit einem dritten Zahnrad gekoppelt sein. Das dritte Zahnrad als Teil des Taumelscheibengetriebes steht mit den beiden Kegelradkränzen der beiden Doppelräder in Eingriff. Die Drehachse D des dritten Zahnrads steht in einem rechten Winkel zu der gemeinsamen Achse A der angetriebenen Doppelräder. Vorteilhaft wird durch die drei miteinander kämmenden Zahnräder jede Bewegung der beiden angetriebenen Zahnräder direkt auf das mit der räumlich verstellbaren Taumelscheibe gekoppelte dritte Zahnrad übertragen, sodass die Stellbewegungen der Antriebe über die auf die Taumelscheibe übertragen werden, die dadurch um die gemeinsame Achse A und Drehachse D verkippt bzw. verschwenkt werden kann.

Ferner kann in einer weiteren Ausführungsform des erfindungsgemäßen Lenkgetriebes die Taumelscheibe mit einem vierten Zahnrad gekoppelt sein, das mit den beiden Kegelradkränzen der beiden Doppelräder gekoppelt und auf der abgewandten Seite von dem dritten Zahnrad angeordnet ist. Hierdurch erfolgt wird die umlaufende Verzahnungskette geschlossen und für eine gleichmäßig umlaufende und spielfreie Kraftverteilung gesorgt.

Die Erfindung bezieht sich ferner auf ein chirurgisches Instrument, das einen Schaft, eine am proximalen Ende des Schaftes angeordnete Betätigungseinheit und ein am distalen Ende des Schaftes angeordnetes Werkzeug auf. Das Werkzeug hat eine Werkzeugspitze, die mittels einer distalen Abwinkelungsmechanik abgewinkelt werden kann. Die Abwinkelungsmechanik kann durch eine mittels zweier Antriebe räumlich ausrichtbare Taumelscheibe gesteuert bzw. ausgerichtet werden, wozu das chirurgische Instrument ein erfindungsgemäßes Lenkgetriebe aufweist, wobei die beiden Antriebe Teil des erfindungsgemäßen Lenkgetriebes sind, das dazu ausgebildet ist, die Stellwinkel der zwei Antriebe auf die räumliche Ausrichtung der Taumelscheibe zu übertragen, um so die Abwinkelungsmechanik zu steuern.

Durch das erfindungsgemäße Lenkgetriebe kann das chirurgische Instrument konstruktiv einfach und platzsparend aufgebaut werden, so dass eine einfache Verbindung zu einem Roboter-Arm ermöglicht werden kann, bei der die Bewegung der Antriebe linear auf die Werkzeugspitze übertragen werden kann. Folge ist eine exakt steuerbare Verwendung des chirurgischen Instruments.

Um die räumlich verstellbare Taumelscheibe trotz der drehfesten Kopplung mit dem dritten Zahnrad, das mit den beiden Kegelradkränzen der beiden Doppelräder in Eingriff steht, dreidimensional verstellen zu können, d. h. die Kipp- bzw. Schwenkbewegungen mit einer Rotation der Taumelscheibe um die Längsachse überlagern zu können, kann eine bevorzugte Ausführungsform des chirurgischen Instruments vorsehen, dass die Taumelscheibe um die Längsachse B des Schaftes über einen Lagerring rotierbar in einem Lenkring gelagert ist, der drehfest mit dem dritten Zahnrad gekoppelt ist. Zur rotativen Kopplung der Taumelscheibe mit einer koaxial zu einer Längsachse B des Schaftes verlaufenden Hauptwelle kann die Taumelscheibe kardanisch mit der Hauptwelle gekoppelt sein. Somit kann die Werkzeugspitze mittels der Taumelscheibe zusätzlich zum Verschwenken bzw. Verkippen durch die beiden Antriebe durch die Hauptwelle relativ zur Längsachse des Schaftes um die Längsachse des Schaftes rotiert werden.

Zur Ausbildung der kardanischen Lagerung der räumlich verstellbaren Taumelscheibe kann eine Ausführungsform des erfindungsgemäßen chirurgischen Instruments vorsehend, dass die Taumelscheibe über zwei um 180 ° versetzt zueinander angeordnete Lagerstifte verschwenkbar auf einer Kreuzgelenkscheibe gelagert ist, wobei die Kreuzgelenkscheibe über zwei um 180 ° versetzt zueinander angeordnete Lagerstifte verschwenkbar auf der Hauptwelle gelagert ist und wobei die Lagerstifte der Taumelscheibe und der Kreuzgelenkscheibe um 90 ° versetzt zueinander angeordnet sind. Die kardanische Aufhängung ermöglicht eine Bewegungsführung in allen drei Raumachsen, wodurch die Werkzeugspitze gezielt gesteuert werden kann. Alternativ zu einer Kreuzgelenkscheibe mit zwei rechtwinklig gekreuzten Stift-Paaren zur kardanischen Lagerung der Taumelscheibe auf der Hauptwelle kann eine vorteilhafte Ausführungsform vorsehen, dass zur kardanischen Lagerung die Hauptwelle zwei in ihrer Außenfläche vorliegende Führungsnuten aufweist, die sich diametral und längs der Hauptwelle erstrecken, wobei die Taumelscheibe, die kreisringförmig mit einer Außenseite und einer Innenseite ausgebildet ist, zwei diametral und radial nach innen weisend an der Taumelscheibe angeordnete Stifte aufweist. Jeder der zwei fest an bzw. in der Taumelscheibe montierten Stifte greift in eine der beidseitig in die Hauptwelle eingebrachten Führungsnuten ein, sodass ein Drehwinkel der Welle auf die Taumelscheibe übertragbar ist. Vorteilhaft ergibt sich so eine drehsteife Verbindung zwischen Hauptwelle und Taumelscheibe, die auch bei einem großen Winkelversatz (± 40 ° und mehr) und Axialversatz eine Drehwinkelübertragung erlaubt, und dabei sehr kompakt aufgebaut, sowie einfach herzustellen und zu montieren ist. Grundsätzlich können allerdings auch eine Bogenzahnkupplung trotz eines relativ geringen Winkelversatzes, ein Gleichlaufgelenk trotz der aufwändigen Fertigung und komplexen Montage oder eine stoffschlüssige Kupplung, die häufig mit einer spielbehafteten Drehwinkelübertragung verbunden ist, zur kardanischen Lagerung einer Taumelscheibe auf einer Hauptwelle eingesetzt werden.

In einer weiteren Ausführungsform des erfindungsgemäßen chirurgischen Instruments verlaufen in Längsrichtung des Schaftes Lenkdrähte, die mit der Taumelscheibe des Lenkgetriebes verbunden sind. Vorzugsweise können die Lenkdrähte an der Taumelscheibe lösbar befestigt sein, beispielsweise mittels einer Klemmverbindung, damit in einem Fall der Beschädigung die Lenkdrähte einfach ausgetauscht werden können. Da die Taumelscheibe rotatorisch mit der Hauptwelle gekoppelt ist und durch den Lagerring drehbar in dem Lenkring gelagert ist, der drehfest mit dem dritten Zahnrad gekoppelt ist, wird bewirkt, dass weiter vorteilhaft zum Verschwenken der Werkzeugspitze relativ zur Längsachse und Rotieren um die Längsachse des Schaftes das Verdrillen der Lenkdrähte verhindert wird.

Vorteilhaft an dieser Konstruktion gegenüber bekannten Konstruktionen ist, dass nicht nur ist die Verwendung einer geringen Anzahl von Lenkdrähten, nämlich von nur vier Lenkdrähten, und eine ausschließlich manuelle Betätigbarkeit der als Antrieb für die Lenkdrähte dienenden räumlich verstellbaren Scheibe möglich ist, sondern dass eine Vielzahl an Lenkdrähten frei gewählt werden und dadurch eine feinfühlige und reproduzierbare Verstellung der distalseitigen Schwenkglieder möglich ist.

Ferner sieht eine noch weitere Ausführungsform des erfindungsgemäßen chirurgischen Instruments vor, dass das vierte Zahnrad mit der Taumelscheibe über einen Lagerring mit dem Lenkring gekoppelt ist, wobei das vierte Zahnrad gegenüber dem dritten Zahnrad frei drehbar ist. Dieses vierte Zahnrad schließt die umlaufende Verzahnungskette und sorgt so für eine umlaufende und spielfreie Kraftverteilung.

In einer weiteren Ausführungsform des erfindungsgemäßen chirurgischen Instruments ist ein Betätigungselement axial verschiebbar in dem Schaft gelagert und steht proximalseitig mit der Betätigungseinheit in Wirkverbindung. Die distale Abwinkelungsmechanik der abwinkelbaren Werkzeugspitze besteht aus an dem distalen Ende des Schaftes angeordneten Schwenkgliedern, die über die in Längsrichtung des Schaftes verlaufenden Lenkdrähte mit der Taumelscheibe des Lenkgetriebes verbunden sind. Die Lenkdrähte können bevorzugt an der Taumelscheibe lösbar mittels einer Klemmverbindung klemmend gelagert, damit in einem Fall der Beschädigung die Lenkdrähte einfach ausgetauscht werden können. Alternativ können die Lenkdrähte beispielsweise auch durch Schweißen oder Crimpen an der Taumelscheibe befestigt sein.

In einer weiteren Ausführungsform des erfindungsgemäßen chirurgischen Instruments ist ein radialer Abstand der Lenkdrähte von der Längsachse des Schaftes an der Taumelscheibe größer als am proximalen Ende des Schaftes, aus dem die Lenkdrähte austreten. Dabei können die Lenkdrähte sich von dem proximalen Ende des Schaftes direkt zu der Taumelscheibe erstrecken, wobei die Lenkdrähte in Bezug auf eine Scheibenfläche der Taumelscheibe einen Winkel kleiner als 90 ° bilden. Alternativ kann distalseitig vor der Taumelscheibe eine Fächerscheibe auf der Hauptwelle angeordnet sein, die den radialen Abstand der aus dem proximalen Schaftende austretenden Lenkdrähte von der Längsachse des Schaftes vergrößert, sodass die Lenkdrähte zwischen der Fächerscheibe und der Taumelscheibe annährend parallel zueinander verlaufen und in Bezug auf eine Scheibenfläche der Taumelscheibe einen Winkel von ca. 90 ° bilden. Aufgrund des geringeren Bauraumbedarfs kann die Variante ohne Fächerscheibe bevorzugt sein. Durch die Vergrößerung des radialen Abstandes der Lenkdrähte von der Längsachse des Schaftes, von beispielsweise einem Durchmesser von 4 mm auf einen Durchmesser von 18 pmm, werden nicht nur die Montage und Fertigung des mit der räumlich verstellbaren Scheibe ausgestatten Antriebs der Lenkdrähte vereinfacht, sondern auch die Verstellwinkel der räumlich verstellbaren Scheibe bzw. infolge des vergrößerten Hebels die zur Abwinkelung benötigten Kräfte verringert, um einen dem Maß der Durchmesservergrößerung entsprechenden Verschwenkwinkel der Werkzeugspitze zu erzielen.

Um beim Verschwenken des dritten und vierten Zahnrads relativ zur Längsachse des Schaftes eine Kollision der Zahnräder mit den Lenkdrähten und gegebenenfalls dem Betätigungselement zu vermeiden, können in den Zahnkränzen des dritten Zahnrads und des vierten Zahnrads Aussparungen für die Lenkdrähte und das Betätigungselement ausgebildet sein.

Das erfindungsgemäße chirurgische Instrument hat den Vorteil, dass viele dünne Lenkdrähte zur Ansteuerung der verschwenkbaren Werkzeugspitze verwendet werden können und diese Ansteuerung aufgrund des motorisierten Antriebs für die räumlich verstellbare Scheibe, an der die Lenkdrähte proximalseitig gelagert sind, feinfühlig, exakt und reproduzierbar erfolgt.

Weitere Ausführungsformen des Lenkgetriebes und des chirurgischen Instruments sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen

Dabei zeigen:
- Fig. 1: eine perspektivische Ansicht des chirurgischen Instruments mit schematisch dargestellter Betätigungseinheit,
- Fig. 2: eine perspektivische Detailansicht einer ersten Ausführungsform des erfindungsgemäßen Lenkgetriebes,
- Fig. 3: eine perspektivische Detailansicht eines Zugmittelantriebs,
- Fig. 4: eine Fig. 2 entsprechende perspektivische Ansicht teilweise ausgeschnitten, und
- Fig. 5: eine perspektivische, teilweise ausgeschnittene Detailansicht einer alternativen Taumelscheibenlagerung.

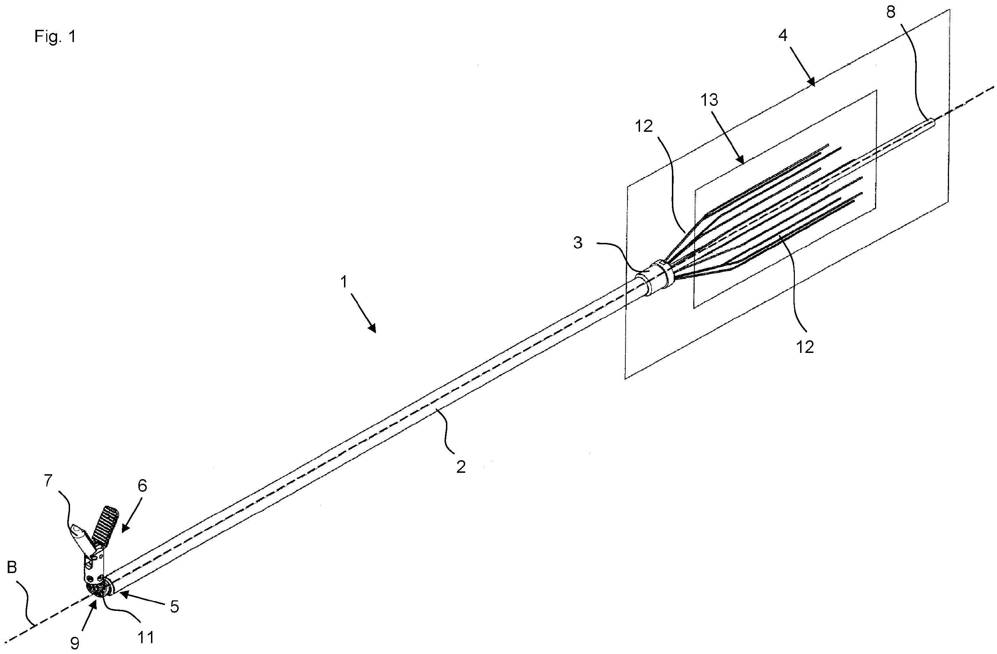

In Fig. 1 ist ein chirurgisches Instrument 1 mit einem hohlen Schaft 2 gezeigt, das eine am proximalen Ende 3 des Schaftes 2 angeordnete, schematisch dargestellte Betätigungseinheit 4 und eine am distalen Ende 5 des Schaftes 2 angeordnete Werkzeugspitze 6 aufweist. Die Werkzeugspitze 6 ist mit einem Instrument 7 verbunden, wobei das Instrument 7 über ein axial verschiebbar im Schaft 2 gelagertes Betätigungselement 8 betätigt werden kann, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln.

Bei dem Instrument 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln.

Die Werkzeugspitze 6 ist über einen Gelenkmechanismus 9 relativ zur Längsachse B des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Antrieb 13 verbunden sind, dass eine Bewegung des proximalseitigen Antriebs 13 eine entsprechende relative Bewegung der distalseitigen Schwenkglieder 11 und somit ein Verschwenken der Werkzeugspitze 6 verursacht.

Auch wenn voranstehend und nachfolgend nur der Begriff Lenkdrähte verwendet wird, können funktional auch Lenkseile verwendet werden, weshalb der Begriff Lenkdrähte hierin synonym auch als Lenkseile zu verstehen ist.

Das axialverschiebbar im Schaft 2 gelagerte Betätigungselement 8 zum Betätigen des beispielsweise aus zwei Maulteilen bestehenden Instruments 7 ist bei den dargestellten Ausführungsformen als Zug- / Schubstange ausgebildet.

Der Antrieb 13 für die Lenkdrähte 12 ist bei dem in den Abbildungen dargestellten und nachfolgend beschriebenen chirurgischen Instrument 1 als motorisierter Antrieb 13 ausgebildet.

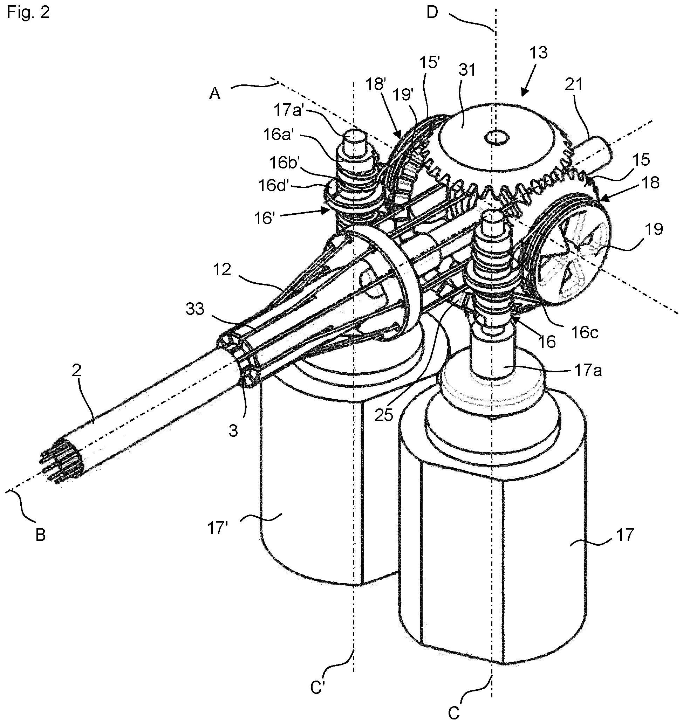

Kernstück des Antriebs 13 ist eine räumlich verstellbare Taumelscheibe 14 (Fig. 2, 4 und 5), an der die Lenkdrähte 12 so gelagert sind, dass eine Verlagerung der Taumelscheibe 14 über die an ihr gelagerten Lenkdrähte 12 ein Verschwenken der Werkzeugspitze 6 bewirkt. Mit dem motorisierten Antrieb 13 kann die Taumelscheibe 14 verlagert werden; mit ihm ist es möglich, die Lenkdrähte 12 zum Verschwenken der distalseitigen Schwenkglieder 11 bzw. der Werkzeugspitze 6 exakt, feinfühlig in kleinsten Schritten und auch reproduzierbar anzusteuern. Darüber hinaus ist die Anzahl der zu verwendenden Lenkdrähte 12 für ein motorisiertes Lenkgetriebe 13 recht frei wählbar.

In Fig. 2, 4 und Fig. 5 ist das Lenkgetriebe 13 vereinfacht dargestellt, wobei das Lenkgetriebe 13 in der Mitte die Taumelscheibe 14 aufweist. Mit der Taumelscheibe 14 sind vier Zahnräder verbunden. Unterhalb und oberhalb der Taumelscheibe 14 sind ein drittes Zahnrad 25 und ein viertes Zahnrad 31 angeordnet und operativ mit der Taumelscheibe 14 gekoppelt. D. h., eine Bewegung eines dieser Zahnräder 25, 31 hat eine direkte Bewegung der Taumelscheibe 14 zur Folge. In die Zahnräder 25, 31 greifen jeweils zu einer linken und rechten Seite angeordneten Doppelräder 18, 18' ein. Hierzu weisen die Doppelräder Kegelradkränze 15, 15' auf, die direkt in die Zahnkränze der Zahnräder 25, 31 eingreifen. Die Doppelräder 18, 18' weisen an voneinander weg weisenden Seiten entlang ihrer Mittelachse A, die auch die gemeinsame Drehachse beider Doppelräder 18, 18' bildet, Zugmittelscheiben 19, 19' auf, die an den Rückseiten der Kegelradkränze 15, 15' angeordnet und fest mit diesen verbunden sind.

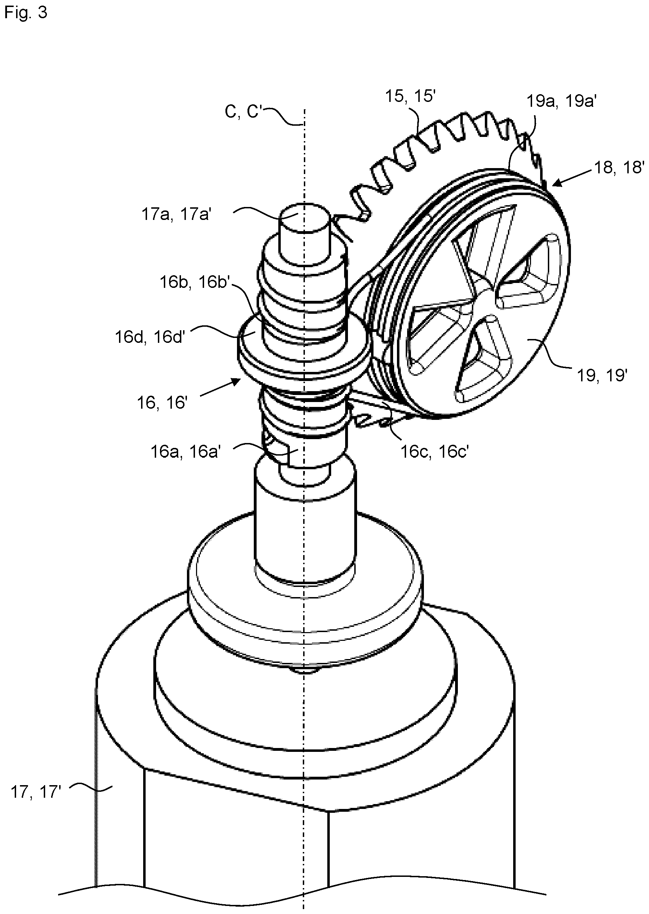

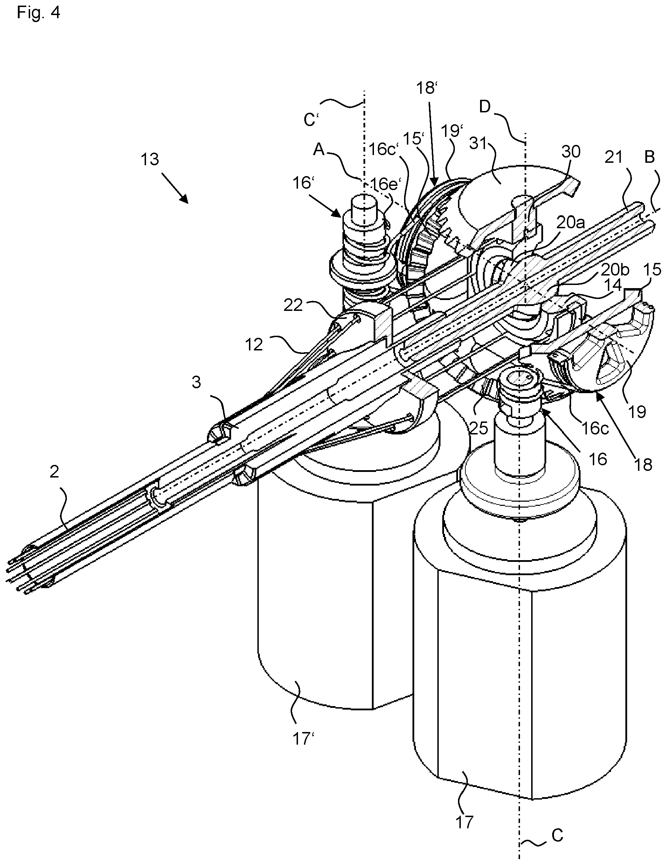

Wie besonders gut in der Detaildarstellung in Fig. 3 zu sehen, hat jede Zugmittelscheibe 19, 19' Nuten 19a, 19a', in denen Zugmittel 16c, 16c', wie bspw. ein Seil oder ein Riemen, geführt sind. Jedes Zugmittel 16c, 16c' verbindet die jeweilige Zugmittelscheibe 19, 19' mit einer Spindel 16a, 16a', die Teil eines Zugmittelantriebs 16, 16' ist. Die Spindeln 16a, 16a' weisen Nuten 16b, 16b' auf, die spiralförmig in den Körper der Spindeln 16a, 16a' eingebracht sind. Damit die Zugmittel 16c, 16c nicht von der Spindel 16a, 16a' rutschen, ist zusätzlich eine Spindelmutter 16d, 16d' vorgesehen, die um die einen mittleren Abschnitt der Spindel 16a, 16a' angeordnet ist. Grundsätzlich kann das Zugmittel durch ein geschlossenes Seil oder einen geschlossenen Riemen bereitgestellt werden, der die Spindel und den Antriebskranz umschlingt. Im dargestellten Beispiel ist das Zugmittel 16c, 16c' ein offenes Seil bzw. Riemen mit zwei Enden, von denen in Fig. 4 ein Zugmittelende 16e' am oberen Ende der hinteren Spindel 16' zu sehen ist. Die Seil- bzw. Riemenenden 16e' sind jeweils in einem durch die Spindelmutter 16d, 16d' begrenzten Nutabschnitt der Spindelnut 16b, 16b' durch Nutensteine (nicht zu sehen) befestigt, während das Zugmittel 16c, 16c' den Antriebskranz 19a, 19a' umschlingt. Selbstverständlich kann alternativ die Spindel durch das Zugmittel umschlungen werden, während die Zugmittelenden durch Nutensteine in der Nut des als Zugmittelscheibe ausgebildeten Antriebskranzes 19a, 19a' befestigt sind. Ferner besteht auch die Möglichkeit, zwei Zugmittel einzusetzen, wobei jeweils ein Zugmittelende in der Spindelnut und das andere in der Zugmittelscheibennut durch Nutensteine befestigt werden.

Angetrieben werden die Spindeln 16a,16a' durch Motoren 17,17', wobei die Spindeln 16a,16a' auf Antriebswellen 17a, 17a' sitzen, die direkt mit den Motoren 17, 17' verbunden sind und die Spindeln 16a, 16a' in Rotation oder Oszillation versetzen. Durch die Betätigung einer Spindel 16a, 16a' wird an einem Ende ein Aufwickeln und am anderen Ende ein Abwickeln des Zugmittels 16c, 16c' auf der Spindel 16a, 16a' bewirkt, wodurch das Zugmittel 16c, 16c' die Antriebsbewegung über den als Zugmittelscheibe ausgeführten Antriebskranz 19a, 19a' auf das Antriebsrad 18, 18' überträgt. Die Drehachse C, C' der Antriebskegelräder 16,16' ist gleich mit der Drehachse der Motoren 17, 17'. In Fig. 2 und 4 sind diese Drehachsen C, C' senkrecht, d. h. rechtwinklig, zu der gemeinsamen Achse A und senkrecht zu der Längsachse B und damit in der dargestellten neutralen Stellung der Taumelscheibe 14 auch parallel zu einer Drehachse D des dritten Zahnrads 25 und des vierten Zahnrads 31.

Selbstverständlich sind auch Anordnungen der Motoren 17, 17' und der Spindeln 16a, 16a' in Wirkverbindung mit den Zugmittelscheiben 19, 19' denkbar, die von den dargestellten Beispielen abweichen. Die Anordnung der Antriebseinheiten aus Motor und Spindel kann entlang des Umfangs des jeweiligen Doppelrads frei gewählt werden, sodass ein vorhandener Bauraum optimal genutzt werden kann, bzw. die Dimensionen der Betätigungseinheit verringert werden können. D. h., die Drehachsen C, C' müssen nicht wie bei einem direkten Antrieb der Kegelräder 15, 15' parallel zur Mittelachse A der Doppelräder 18, 18' oder zur Längsachse B des Instruments sein, sondern können theoretisch in beliebiger Orientierung und auch unabhängig voneinander an den Doppelrädern 18, 18' angeordnet sind. Allerdings kann das mit Fig. 2 bis 4 dargestellte Anordnungsbeispiel aus Konstruktionsgründen bevorzugt sein, da durch die zur Drehachse D parallele Anordnung der Motoren 17, 17' nebeneinander nach Fig. 2 bis 4 die Bauhöhe reduziert wird, obwohl dies auch durch eine zur Längsachse B parallele Anordnung der Motoren 17, 17' erreicht wird. Allerdings ist auch denkbar, dass eine der Antriebseinheiten aus Motor und Spindel um 180 ° in Bezug auf die Doppelräder 18, 18' versetzt wird, sodass die Motor-Spindel-Zugmittelscheibe-Anordnungen diametral versetzt sind und in entgegen gesetzte Richtungen weisen, insbesondere in einer zur Längsachse B parallelen Anordnung der Motoren 17, 17', da hierdurch die Bauhöhe nicht vergrößert wird.

Angetrieben werden die Spindeln 16a, 16a' durch Motoren 17, 17' über die Antriebswellen 17a, 17a' der Motoren 17, 17', deren Drehachse einer Drehachse C, C' der Motoren 17, 17' entspricht. Durch Drehen der Spindel 16a, 16a', in deren spiralförmige Nut 16b, 16b' die Zugmittel 16c, 16c' geführt sind, wird das Zugmittel 16c, 16c' an einem Ende der Spindel 16a, 16a' abgewickelt und an dem anderen Ende der Spindel 16a, 16a' aufgewickelt, so das ein gerichteter Zug über das Zugmittel 16c, 16c' auf die Zugmittelscheibe 19,19' entsteht und diese in Rotation oder Oszillation versetzt werden kann. So wird die Rotation der Motoren 17,17' um ihre Drehachse C, C' und damit der Spindeln 16a, 16a' auf die Doppelräder 18, 18' um deren Drehachse A übertragen. Die Drehbewegung der Doppelräder 18, 18' bewirkt dann eine Drehbewegung des dritten Zahnrads 25 bzw. des vierten Zahnrads 31 um deren Drehachse D, die im rechten Winkel zu der gemeinsamen Achse A der Doppelräder 18, 18' liegt, und damit eine Bewegung der Taumelscheibe 14. In Fig. 2 sind diese Drehachsen C, C' parallel zu einer Drehachse D des dritten Zahnrads 25 und des vierten Zahnrads 31. In Fig. 3 sind die Antriebsachsen C, C' parallel zu der Instrumentenachse B und damit im rechten Winkel zu der Drehachse D des dritten Zahnrads 25 und des vierten Zahnrads 31 sowie der gemeinsamen Drehachse A der Doppelräder 18, 18' angeordnet.

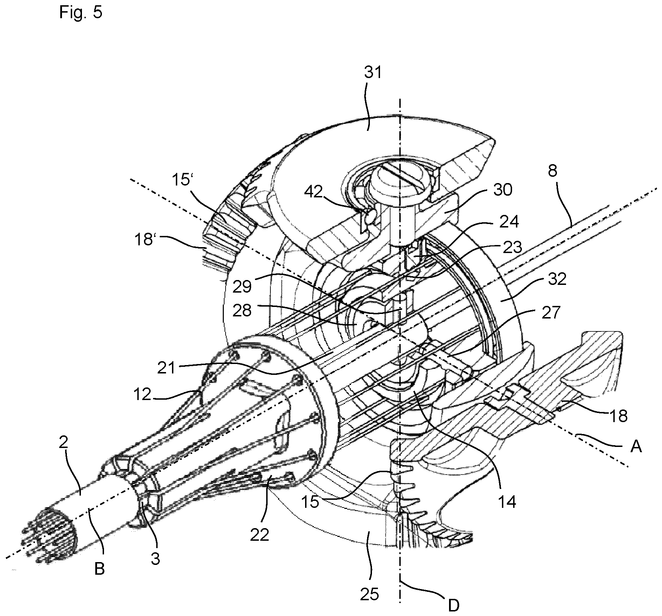

Der Aufbau und der Betrieb des Lenkgetriebes 13 in Bezug auf die Ansteuerung der über die Antriebseinheiten betätigbaren Taumelscheibe 14 und deren Lagerung werden nachfolgend anhand Fig. 4 und 5 beschrieben, wobei in Fig. 5 die Kegelräder mit den Kegelradkränzen 15, 15' der Doppelräder 18, 18' dargestellt sind.

Im Schaft 2 des Instruments 1 ist eine sich koaxial zur Längsachse B des Schaftes 2 erstreckende hohle Hauptwelle 21 angeordnet, die um die Längsachse B des Schaftes 2 rotierbar ist und sich über das proximale Ende 3 des Schaftes 2 hinaus bis in den Bereich des Lenkgetriebes 13 erstreckt. Innerhalb dieser hohlen Hauptwelle 21 ist axialverschieb-bar das Betätigungselement 8 zur Betätigung des Instruments 7 gelagert.

Die Lenkdrähte 12, die am proximalen Ende 3 des Schaftes 2 aus dem Schaft 2 austreten, wofür am proximalen Schaftende ein Schaftendstück 3 vorgesehen sein kann, in dem Durchtrittschlitze 33 für die Lenkdrähte 12 vorgesehen sind, werden in den dargestellten Beispielen über eine drehfest in Bezug auf die Hauptwelle 21 an dem Schaftendstück 3 angeordnete Fächerscheibe 22 aufgefächert, wodurch der radiale Abstand der Lenkdrähte 12 von der Längsachse B des Schaftes 2 vergrößert wird. Während der Durchmesser des die Längsachse B des Schaftes 2 koaxial umgebenden Bündels der Lenkdrähte 12 innerhalb des Schaftes 2 bzw. an dem distalen Ende 5 im Bereich der Abwinkelungsmechanik 9 beispielsweise 4 mm beträgt, beträgt der Durchmesser des von den Lenkdrähten 12 gebildeten Bündels hinter der Fächerscheibe 22 beispielsweise 18 mm. Bei dem dargestellten Ausführungs-beispiel besteht das Bündel aus zehn einzelnen Lenkdrähten 12. Durch die mit Hilfe der Fächerscheibe 22 erzielte Vergrößerung des radialen Abstandes der Lenkdrähte 12 von der Längsachse B des Schaftes 2 werden nicht nur die Montage und Fertigung des mit der Taumelscheibe 14 ausgestatten Getriebes 13 vereinfacht, sondern wird auch der notwendige Verstellwinkel der Taumelscheibe 14 proportional verringert, um einen erwünscht hohen Verschwenkwinkel der Werkzeugspitze 6 zu erzielen. Mit der beispielhaft beschriebenen Vergrößerung des Durchmessers des Lenkdrahtbündels von 4 mm innerhalb des Schaftes 2 auf 18 mm hinter der Fächerscheibe 22 verringert sich der Verstellwinkel der Taumelscheibe 14 entsprechend um das 4,5-fache gegenüber dem am distalen Ende erzielbaren Verschwenkwinkel der Werkzeugspitze 6. Um diese um 90 °abzuwinkeln, bedarf es somit nur einer Verschwenkung der Taumelscheibe 14 um 20 °.

Proximalseitig hinter der Fächerscheibe 22 werden die parallel zur Längsachse B des Schaftes 2 verlaufenden Lenkdrähte 12 der Taumelscheibe 14 zugeführt. In einer nicht dargestellten Alternative verlaufen können die am proximalen Ende 3 austretenden Lenkdrähte 12 direkt ohne Fächerscheibe zur Taumelscheibe 14 verlaufen, sodass die Lenkdrähte unter einem Winkel zur Längsachse B der Taumelscheibe 14 zugeführt werden. Zum Festlegen der Lenkdrähte 12 an der Taumelscheibe 14 sind in der Taumelscheibe 14 Durchgangs-bohrungen 23 für jeden Lenkdraht 12 ausgebildet, wobei im dargestellten Beispiel die Lenkdrähte 12 innerhalb der Durchgangsbohrungen 23 über Madenschrauben 24 kraft-schlüssig mit der Taumelscheibe 14 verbunden und fixiert sind. Dazu alternative Befestigungsformen der Lenkdrähte an der Taumelscheibe umfassen beispielsweise auch Schweißen oder Crimpen oder andere Klemmvorrichtungen.

Die Doppelräder 18, 18' als Antriebsräder sind mit dem dritten Zahnrad 25 gekoppelt, das vorzugsweise als Kegelrad ausgebildet ist und mit den beiden Kegelradkränzen 15, 15' der Doppelräder 18, 18' in Eingriff steht, sodass die Drehachse D des dritten Zahnrads 25 die gemeinsame Drehachse A der Doppelräder 18 und 18' sowie die Längsachse B des Schaftes 2 schneidet. Durch die drei miteinander kämmenden Zahnräder 18, 18' und 25 wird jede Bewegung der beiden Doppelräder 18, 18' direkt auf die mit dem dritten Zahnrad 25 gekoppelte Taumelscheibe 14 übertragen, was eine direkte Betätigung der Lenkdrähte 12 bewirkt.

Zur Ausbildung einer kardanischen Lagerung der Taumelscheibe 14 auf der Hauptwelle 21 ist die Taumelscheibe 14 über zwei um 180 ° versetzt zueinander angeordnete Lagerstifte 27 verschwenkbar auf einer Kreuzgelenkscheibe 28 gelagert, die wiederrum über zwei um 180 ° versetzt zueinander angeordnete Lagerstifte 29 verschwenkbar auf der Hauptwelle 21 gelagert ist. In Fig. 5 ist aufgrund der Teilschnittansicht jeweils nur ein Lagerstift 27 und ein Lagerstift 29 zu sehen.

Die Lagerstifte 27 der Taumelscheibe 14 und die Lagerstifte 29 der Kreuzgelenkscheibe 28 sind dabei um 90 ° versetzt zueinander angeordnet. Diese Lagerung ermöglicht es, die Taumelscheibe 14 um zwei rechtwinklig zueinander stehende Achsen relativ zur Längsachse B des Schaftes 2 zu verschwenken und eine Rotation der Hauptwelle 21 um die Längsachse B auf die Taumelscheibe 14 zu übertragen, wodurch über die Lenkdrähte 12 distalseitig die Werkzeugspitze 6 (vgl. Fig. 1) in alle Raumrichtungen relativ zur Längsachse B des Schaftes 2 verschwenkbar ist.

Eine alternative kardanische Lagerung der Taumelscheibe 14 auf der Hauptwelle 21 weist das in Fig. 4 dargestellte Lenkgetriebe 13 auf. Diese konstruktiv einfachere, kompakter aufgebaut und einfacher zu montiere Lagerungsanordnung ermöglicht es ebenfalls, die Taumelscheibe 14 um zwei Freiheitsgrade zu verschwenken und um die Längsachse B zu rotieren, wodurch über die Lenkdrähte 12 distalseitig die Werkzeugspitze 6 in alle Raumrichtungen relativ zur Längsachse B des Schaftes 2 verschwenkbar ist. Dabei weist die Hauptwelle 21 in dem zur Lagerung der Taumelscheibe 14 vorgesehenen Bereich zwei sich längs der Hauptwelle 21 erstreckende und beidseitig bzw. diametral in der Hauptwelle 21 eingebrachte Führungsnuten 20a auf, in die zwei diametral und radial nach innen weisend an der Taumelscheibe 14 angeordnete Stifte 29 eingreifen, wobei in Fig. 4 nur eine Führungsnut 20a mit dem der darin eingreifenden Stift 29 zu sehen ist. Durch diesen Eingriff kann die Taumelscheibe 14 aus einer neutralen Position, in der die Taumelscheibe 14 in einer durch die Drehachse A definierten Ebene senkrecht, d. h. rechtwinklig, zur Längsachse B liegt, sowohl um Drehachse D als auch um Drehachse A verschwenkt werden. Überlagerte Bewegungen durch Verschwenken um beide Drehachsen A, D, sind ebenfalls möglich. Ferner gestattet der Eingriff der Stifte 29 in die Führungsnuten 20a die Übertragung eines Drehwinkels der Hauptwelle 21 auf die Taumelscheibe 14, sodass die Taumelscheibe 14 dreidimensional relativ zur Längsachse B des Schaftes 2 verlagert werden kann. Die maximale Verkippung bzw. Verdrehung bzw. die maximalen Kipp- und Drehwinkel um Drehachse A und D sind durch die Länge und Tiefe der Führungsnuten 20a im Zusammenwirken mit Innendurchmesser und Stärke der Taumelscheibe 14 und Länge der Stifte 29 bestimmt. Im dargestellten Beispiel weist die Hauptwelle 21 in dem zur Lagerung der Taumelscheibe 14 vorgesehenen Bereich einen Kugelabschnitt 20b auf, an dem die Führungsnuten 20a vorliegen und der die Taumelscheibe 14 in axialer Richtung festlegt. Die Taumelscheibe 14 weist hierbei eine an den Kugelabschnitt 20b angepasst konturierte Aufnahmeausnehmung auf.

Wie weiterhin aus Fig. 4 und 5 ersichtlich, ist die räumlich verstellbare Taumelscheibe 14 in einem drehfest mit dem dritten Zahnrad 25 gekoppelten Lenkring 30 gelagert. Um die durch die Doppelräder 18, 18' und Zahnrad 25 gebildete Verzahnungskette zu einem geschlossenen Verzahnungsring zu schließen, der eine gleichmäßig umlaufende Kraftverteilung gewährleistet, ist auf der Drehachse D des dritten Zahnrads 25 gegenüber liegend zum dritten Zahnrad 25 das vierte Zahnrad 31 angeordnet, das ebenfalls vorzugsweise als Kegelrad ausgebildet ist mit den Kegelradkränzen 15, 15' der beiden Doppelräder 18 und 18' in Eingriff steht.

Die Taumelscheibe 14 ist über einen Lagerring 32 in dem drehfest mit dem dritten Zahnrad 25 gekoppelten Lenkring 30 gelagert, um eine Rotation der Taumelscheibe 14 um die Längsachse B des Schaftes 2 zu ermöglichen. Der drehfest mit dem dritten Zahnrad 25 gekoppelte Lenkring 30 ist durch eine Lagerung mittels Lagerring 42 frei in Bezug auf das vierte Zahnrad 31 drehbar, so dass eine Rotation des vierten Zahnrads 31 um seine Drehachse D keine Verdrehung des Lenkrings 30 und der Taumelscheibe 14 bewirkt.

Die beschriebene kardanische Lagerung der Taumelscheibe 14 auf der Hauptwelle 21 ermöglicht es, die Taumelscheibe 14 dreidimensional relativ zur Längsachse B des Schaftes 2 zu verlagern. Wenn ausgehend von der in Fig. 4 und 5 dargestellten neutralen Ausgangsstellung, in der die Taumelscheibe 14 senkrecht zur Längsachse B des Schaftes 2 ausgerichtet ist, die Doppelräder 18, 18' über die Motoren 17, 17'so angetrieben werden, dass sich die Doppelräder 18, 18' in dieselbe Richtung drehen, bewirkt diese Verdrehung der Doppelräder 18, 18' aufgrund des kämmenden Eingriffs mit dem dritten Zahnrad 25 und dem vierten Zahnrad 31 ein Verkippen der Baueinheit, die aus dem dritten Zahnrad 25, der mit dem dritten Zahnrad 25 über den Lenkring 30 gekoppelten Taumelscheibe 14 und dem vierten Zahnrad 31 gebildet wird, um die gemeinsame Drehachse A der Doppelräder 18, 18'.Zur Vereinfachung der Funktionsbeschreibung wird nachfolgend auf die Ausrichtung der Lagerstifte 27, 29 der kardanischen Lagerung in Bezug auf die Drehachsen A und D genommen. Tatsächlich werden bei Rotation der Hauptwelle 21 und damit der Taumelscheibe 14 die Lagerstifte 27, 29 nicht mehr wie dargestellt mit den Achsen A und D fluchten, sodass die durch die Lagerstifte 27, 29 bereitgestellten Schwenkachsen der Taumelscheibe 14 von den Drehachsen A, D des Lenkgetriebes 13 abweichen können.

Im Beispiel von Fig. 5 ermöglichen die mit der Drehachse A der Doppelräder 18, 18' fluchtenden Lagerstifte 27, über die die Taumelscheibe 14 verschwenkbar an der Kreuzgelenkscheibe 28 gelagert ist, dieses Verkippen der Taumelscheibe 14 relativ zur Hauptwelle 21. Dieses Ver-kippen der Taumelscheibe 14 um die Drehachse A relativ zur Längsachse B des Schaftes 2 bewirkt über die Lenkdrähte 12, dass distalseitig die Werkzeugspitze 6 in entsprechender Weise relativ zur Längsachse B des Schaftes 2 verschwenkt wird. Im Beispiel von Fig. 4 wird das Verkippen der Taumelscheibe 14 um die Drehachse A durch die Bewegung der Führungsstifte 29 in den Führungsnuten 20a im Kugelabschnitt 20b der Hauptwelle 21 ermöglicht
Wenn ausgehend von der in Fig. 4 und 5 dargestellten neutralen Ausgangsstellung, in der die Taumelscheibe 14 senkrecht zur Längsachse B des Schaftes 2 ausgerichtet ist, die Doppelräder 18, 18' über die Motoren 17, 17' so angetrieben werden, dass sich die Doppelräder 18, 18' in entgegengesetzte Richtungen drehen, bewirkt diese Verdrehung beider Doppelräder 18, 18' aufgrund des kämmenden Eingriffs mit dem dritten Zahnrad 25 ein Verdrehen der Baueinheit, die aus dem dritten Zahnrad 25, und der mit dem dritten Zahnrad 25 über den Lenkring 30 gekoppelten Taumelscheibe 14 gebildet wird, um die Drehachse D des dritten Zahnrads 25.

Im Beispiel von Fig. 5 ermöglichen die mit der Drehachse D des dritten Zahnrads 25 fluchtenden Lagerstifte 29, über die die Kreuzgelenkscheibe 28 verschwenkbar an der Hauptwelle 21 gelagert ist, zusammen mit der freien Drehbarkeit der Taumelscheibe 14 relativ zum vierten Zahnrad 31 aufgrund des Lagerring 32 dieses Verdrehen der Kreuzgelenkscheibe 28 relativ zur Hauptwelle 21. Dieses Verdrehen der Taumelscheibe 14 um die Drehachse D relativ zur Längsachse B des Schaftes 2 bewirkt über die Lenkdrähte 12, dass distalseitig die Werkzeugspitze 6 in entsprechender Weise relativ zur Längsachse B des Schaftes 2 verschwenkt wird. Analog wird im Beispiel von Fig. 4 das Verdrehen der Taumelscheibe 14 um die Drehachse D durch die in die Führungsnuten 20a eingreifenden und mit der Drehachse D des dritten Zahnrads 25 fluchtenden Stifte 29 bewirkt.

Es ist selbstverständlich möglich, die beschriebenen Bewegungen zu überlagern, sodass beispielsweise die Taumelscheibe 14 um die gemeinsame Drehachse A der Doppelräder 18, 18' verkippt und gleichzeitig auch noch um die Drehachse D des dritten Zahnrads 25 verdreht wird. Durch die Kombination der beiden Bewegungsabläufe aufgrund der einzeln ansteuerbaren Motoren 17, 17' des Getriebes 13 und die Kopplung mit der Hauptwelle 21 lässt sich die Taumelscheibe 14 dreidimensional relativ zur Längsachse B des Schaftes 2 verstellen, woraus aufgrund der Kopplung über die Lenkdrähte 12 eine entsprechende räumliche Verlagerung der Werkzeugspitze 6 resultiert.

Ein wie zuvor beschrieben ausgebildetes chirurgisches Instrument 1 zeichnet sich dadurch aus, dass viele dünne Lenkdrähte 12 zur Ansteuerung der verschwenkbaren Werkzeugspitze 6 verwendet werden können, und diese Ansteuerung auf Grund des motorisierten Antriebs 13 für die Taumelscheibe 14, an der die Lenkdrähte 12 gelagert sind, feinfühlig, exakt und reproduzierbar erfolgt.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, im Umfang der beigefügten Ansprüche. Die vorliegende Erfindung stellt ein Lenkgetriebe 13 für ein chirurgisches Instrument 1 und das chirurgische Instrument 1 selbst bereit. Das Lenkgetriebe 13 ist am proximalen Ende 3 eines Schafts 2 angeordnet, der eine Längsachse B definiert und am distalen Ende 5 eine Abwinkelungsmechanik 9 aufweist. Dabei weist es zwei motorisierte Antriebe auf und ist dazu ausgebildet, über deren Stellwinkel eine Taumelscheibe 14 räumlich auszurichten, die die distale Abwinkelungsmechanik 9 des chirurgischen Instruments 1 steuert. Der erste Antrieb hat eine von einem ersten Motor 17 angetriebene erste Antriebswelle 17a, die mit einer ersten Zugmittelscheibe 19 eines ersten Antriebsrads 18 über ein erstes Zugmittel 16c in Wirkverbindung steht, das zwischen der ersten Zugmittelscheibe 19 und einer ersten Spindel 16a angeordnet ist, die auf der ersten Antriebswelle 17a sitzt, die eine erste Antriebsachse C definiert. Der zweite Antrieb hat eine von einem zweiten Motor 17' angetriebene zweite Antriebswelle 17a', die mit einer zweiten Zugmittelscheibe 19' eines zweiten Antriebsrads 18' über ein zweites Zugmittel 16c' in Wirkverbindung steht, das zwischen der zweiten Zugmittelscheibe 19' und einer zweiten Spindel 16a' angeordnet ist, die auf der zweiten Antriebswelle 17a' sitzt, die eine zweite Antriebsachse C' definiert. Dabei sind das erste und das zweite Antriebsrad 18, 18' als Doppelräder 18, 18' ausgebildet und weisen jeweils die entsprechende Zugmittelscheibe 19, 19' und einen an der jeweiligen rückwärtigen Seite vorliegenden Kegelradkranz 15, 15' auf, wobei zwischen den zwei Antriebsrädern 18, 18', die eine gemeinsame Drehachse A haben, die Taumelscheibe 14 angeordnet ist und die Kegelradkränze 15, 15' einander zugewandt auf der Drehachse A angeordnet sind.

### BEZUGSZEICHENLISTE

- 1: Chirurgisches Instrument
- 2: Schaft
- 3: proximales Ende (Schaft) / Schaftendstück
- 4: Betätigungseinheit
- 5: distales Ende (Schaft)
- 6: Werkzeugspitze
- 7: Instrument
- 8: Betätigungselement
- 9: Abwinkelungsmechanik
- 11: Schwenkglied
- 12: Lenkdraht
- 13: Lenkgetriebe
- 14: Taumelscheibe
- 15, 15': Kegelradkranz des jew. Doppelrades 18,18'
- 16, 16': Zugmittelantrieb
- 16a, 16a': Spindel des Seil- / Riemenantriebs 16, 16'
- 16b, 16b': Nut der Spindel 16a, 16a'
- 16c, 16c': Seil / Riemen des Seil-/Riemenantrieb 16, 16'
- 16d, 16d': Spindelmutter des Seil- / Riemenantriebs 16, 16'
- 16e, 16e': Seil- / Riemenende
- 17, 17': Motor
- 17a, 17a': Antriebswelle
- 18, 18': Doppelrad
- 19, 19': Zugmittelscheibe des jew. Doppelrades 18,18'
- 19a, 19a': Nut Zugmittelscheibe
- 20a, 20b: Führungsnut, Kugelabschnitt
- 21: Hauptwelle
- 22: Fächerscheibe
- 23: Durchgangsbohrungen
- 24: Madenschrauben
- 25: drittes Zahnrad
- 27: Lagerstifte
- 28: Kreuzgelenkscheibe
- 29: Lagerstifte
- 30: Lenkring
- 31: viertes Zahnrad
- 32: Lagerring
- 33: Durchtrittschlitz
- 42: Lagerring
- A: Mittelachse der angetriebenen Doppelzahnräder 18,18
- B: Längsachse des Instruments bzw. des Schafts
- C: Antriebsachse des ersten Antriebs 17
- C': Antriebsachse des zweiten Antriebs 17'
- D: Drehachse drittes und viertes Zahnrad

## Patentansprüche

1. Lenkgetriebe (13) für ein chirurgisches Instrument (1), das am proximalen Ende (3) eines Schafts (2) angeordnet ist, der eine Längsachse (B) definiert und am distalen Ende (5) eine Abwinkelungsmechanik (9) aufweist,
wobei das Lenkgetriebe (13) zwei motorisierte Antriebe aufweist und dazu ausgebildet ist, über die Stellwinkel der zwei Antriebe eine Taumelscheibe (14) räumlich auszurichten, die dazu ausgebildet ist, die distale Abwinkelungsmechanik (9) des chirurgischen Instruments (1) zu steuern,
wobei
der erste Antrieb eine von einem ersten Motor (17) angetriebene erste Antriebswelle (17a) aufweist und der zweite Antrieb eine von einem zweiten Motor (17') angetriebene zweite Antriebswelle (17a') aufweist, **dadurch gekennzeichnet, dass** die erste Antriebswelle (17a) mit einer ersten Zugmittelscheibe (19) eines ersten Antriebsrads (18) über ein erstes Zugmittel (16c) in Wirkverbindung steht, das zwischen der ersten Zugmittelscheibe (19) und einer ersten Spindel (16a) angeordnet ist, die auf der ersten Antriebswelle (17a) sitzt, die eine erste Antriebsachse (C) definiert, und
die zweite Antriebswelle (17a') mit einer zweiten Zugmittelscheibe (19') eines zweiten Antriebsrads (18') über ein zweites Zugmittel (16c') in Wirkverbindung steht, das zwischen der zweiten Zugmittelscheibe (19') und einer zweiten Spindel (16a') angeordnet ist, die auf der zweiten Antriebswelle (17a') sitzt, die eine zweite Antriebsachse (C') definiert,
wobei das erste und das zweite Antriebsrad (18, 18') als Doppelräder (18, 18') ausgebildet sind und jeweils die entsprechende Zugmittelscheibe (19, 19') und einen an der jeweiligen rückwärtigen Seite vorliegenden Kegelradkranz (15, 15') aufweisen, und wobei
zwischen den zwei Antriebsrädern (18, 18'), die eine gemeinsame Drehachse (A) aufweisen, die Taumelscheibe (14) angeordnet ist und die Kegelradkränze (15, 15') einander zugewandt auf der Drehachse (A) angeordnet sind.

2. Lenkgetriebe (13) nach Anspruch 1,
**dadurch gekennzeichnet, dass**
das erste Zugmittel (16c) und das zweite Zugmittel (16c') geschlossene oder nicht geschlossene Riemen oder Seile sind.

3. Lenkgetriebe (13) nach Anspruch 1 oder 2,
**dadurch gekennzeichnet, dass**
eine Antriebsachse (C) des ersten Motors (17) parallel zu einer Antriebsachse (C') des zweiten Motors (17') vorliegt.

4. Lenkgetriebe (13) nach zumindest einem der Ansprüche 1 bis 3,
**dadurch gekennzeichnet, dass**
die Spindel (16a, 16a') eine umlaufende Nut (16b, 16b') aufweist, in der das Zugmittel (16c, 16c') geführt ist.

5. Lenkgetriebe (13) nach zumindest einem der Ansprüche 1 bis 4,
**dadurch gekennzeichnet, dass**
die Spindel (16a, 16a') eine Spindelmutter (16d, 16d') aufweist.

6. Lenkgetriebe (13) nach zumindest einem der Ansprüche 1 bis 5,
**dadurch gekennzeichnet, dass**
die Zugmittelscheibe (19, 19') eine umlaufende Nut (19a, 19a') aufweist, in der das Zugmittel (19c, 19c') anliegt, und / oder
die Zugmittelscheibe (19, 19') eine umlaufende Nut (19a, 19a') aufweist, in der die Enden des nicht geschlossenen Zugmittels (19c, 19c') mit Nutensteinen befestigt sind.

7. Lenkgetriebe (13) nach zumindest einem der Ansprüche 1 bis 6,
**dadurch gekennzeichnet, dass**
jeder der Motoren (17, 17') über seine jeweilige Spindel (16a, 16a') in beliebiger Position radial von dem jeweiligen Antriebsradkranz (19, 19') weg weisend anordenbar ist, wobei in einer bevorzugten Anordnung die zwei Antriebsachsen (C, C') parallel zueinander verlaufen.

8. Lenkgetriebe (13) nach zumindest einem der Ansprüche 1 bis 7,
**dadurch gekennzeichnet, dass**
die Taumelscheibe (14) mit einem dritten Zahnrad (25) gekoppelt ist, das mit den beiden Kegelradkränzen (15, 15') der beiden Doppelräder (18, 18') in Eingriff steht und dessen Drehachse (D) in einem rechten Winkel zu der gemeinsamen Achse (A) der angetriebenen Doppelräder (18, 18') steht, und / oder
die Taumelscheibe (14) mit einem vierten Zahnrad (31) gekoppelt ist, das mit den beiden Kegelradkränzen (15, 15') der beiden Doppelräder (18, 18') gekoppelt und auf der abgewandten Seite von dem dritten Zahnrad (25) angeordnet ist.

9. Chirurgisches Instrument (1), das einen Schaft (2), eine am proximalen Ende (3) des Schaftes (2) angeordnete Betätigungseinheit (4) und ein am distalen Ende (5) des Schaftes (2) angeordnetes Instrument (7) mit einer mittels einer distalen Abwinkelungsmechanik (9) abwinkelbaren Werkzeugspitze (6), die durch eine mittels zweier Antriebe räumlich ausrichtbare Taumelscheibe (14) steuerbar ist, **dadurch gekennzeichnet, dass**
das chirurgische Instrument (1) ein Lenkgetriebe (13) nach zumindest einem der Ansprüche 1 bis 8 aufweist, das die zwei Antriebe aufweist und dazu ausgebildet ist, die Stellwinkel der zwei Antriebe auf die räumliche Ausrichtung der Taumelscheibe (14) zu übertragen.

10. Chirurgisches Instrument (1) nach Anspruch 9,
**dadurch gekennzeichnet, dass**
die Taumelscheibe (14) zur Kopplung mit einem dritten Zahnrad (25), das mit den beiden Kegelradkränzen (15, 15') der beiden Doppelräder (18, 18') in Eingriff steht, um die Längsachse (B) des Schaftes (2) über einen Lagerring (32) rotierbar in einem Lenkring (30) gelagert ist, der drehfest mit dem dritten Zahnrad (25) gekoppelt ist, wobei die Taumelscheibe (14) kardanisch kardanisch mit einer koaxial zu einer Längsachse (B) des Schaftes (2) verlaufenden Hauptwelle (21) gekoppelt ist.

11. Chirurgisches Instrument (1) nach Anspruch 9 oder 10,
**dadurch gekennzeichnet, dass**
die Taumelscheibe (14) über zwei um 180 ° versetzt zueinander angeordnete Lagerstifte (27) verschwenkbar auf einer Kreuzgelenkscheibe (28) gelagert ist, wobei die Kreuzgelenkscheibe (28) über zwei um 180 ° versetzt zueinander angeordnete Lagerstifte (29) verschwenkbar auf der Hauptwelle (21) gelagert ist und wobei die Lagerstifte (27, 29) der Taumelscheibe (14) und der Kreuzgelenkscheibe (28) um 90 ° versetzt zueinander angeordnet sind,
oder
die kardanische Lagerung durch zwei sich längs erstreckende, diametral in der Hauptwelle (21) vorliegende Führungsnuten (20a) und zwei diametral und radial nach innen weisend an der Taumelscheibe (14) angeordnete Stifte (29) bereitgestellt wird, wobei jeder Stift (29) in eine der Führungsnuten (20a) eingreift, sodass ein Drehwinkel der Hauptwelle (21) auf die Taumelscheibe (14) übertragbar ist.

12. Chirurgisches Instrument (1) nach zumindest einem der Ansprüche 9 bis 11, das vierte Zahnrad (31) mit der Taumelscheibe (14) über einen Lagerring (42) mit dem Lenkring (30) gekoppelt ist, wobei das vierte Zahnrad (31) gegenüber dem dritten Zahnrad (25) frei drehbar ist.

13. Chirurgisches Instrument (1) nach zumindest einem der Ansprüche 9 bis 12,
**dadurch gekennzeichnet, dass**
ein Betätigungselement (8) axial verschiebbar in dem Schaft (2) gelagert ist und proximalseitig mit der Betätigungseinheit (4) in Wirkverbindung steht, und dass die distalen Abwinkelungsmechanik (9) der abwinkelbaren Werkzeugspitze (6) aus an dem distalen Ende (5) des Schaftes (2) angeordneten Schwenkgliedern (11) besteht, die über in Längsrichtung des Schaftes (2) verlaufende Lenkdrähte (12) mit dem Lenkgetriebe (13) verbunden sind.

14. Chirurgisches Instrument (1) nach zumindest einem der Ansprüche 9 bis 13,
**dadurch gekennzeichnet, dass**
ein radialer Abstand der Lenkdrähte (12) von der Längsachse (B) des Schaftes (2) an der Taumelscheibe (14) größer ist als am proximalen Ende (3) des Schaftes (2), aus dem die Lenkdrähte (12) austreten, wobei
- die Lenkdrähte (12) sich von dem proximalen Ende (3) des Schaftes (2) direkt zu der Taumelscheibe (14) erstrecken, wobei die Lenkdrähte (12) in Bezug auf eine Scheibenfläche der Taumelscheibe (14) einen Winkel kleiner als 90 ° bilden,
oder
- distalseitig vor der Taumelscheibe (14) eine Fächerscheibe (22) auf der Hauptwelle (21) angeordnet ist, die den radialen Abstand der Lenkdrähte (12) von der Längsachse (B) des Schaftes (2) vergrößert, sodass die Lenkdrähte (12) zwischen der Fächerscheibe (22) und der Taumelscheibe (14) parallel zueinander verlaufen und in Bezug auf eine Scheibenfläche der Taumelscheibe (14) einen Winkel von 90 ° bilden.

## Claims

1. A steering gear (13) for a surgical instrument (1) which is arranged at the proximal end (3) of a shaft (2), which defines a longitudinal axis (B) and has a bending mechanism (9) at the distal end (5),
wherein the steering gear (13) has two motorised drives and is designed to spatially align a swash plate (14) via the adjustment angles of the two drives, which swash plate is designed to control the distal bending mechanism (9) of the surgical instrument (1),
wherein
the first drive has a first drive shaft (17a) driven by a first motor (17) and the second drive has a second drive shaft (17a') driven by a second motor (17'), **characterised in that** the first drive shaft (17a) is operatively connected to a first traction pulley (19) of a first drive wheel (18) via a first traction means (16c) which is arranged between the first traction pulley (19) and a first spindle (16a) which is seated on the first drive shaft (17a), which defines a first drive axis (C), and
the second drive shaft (17a') is operatively connected to a second traction pulley (19') of a second drive wheel (18') via a second traction means (16c'), which is arranged between the second traction pulley (19') and a second spindle (16a'), which is seated on the second drive shaft (17a'), which defines a second drive axis (C'),
wherein the first and second drive wheels (18, 18') are designed as double wheels (18, 18') and each have the corresponding traction pulley (19, 19') and a bevel gear rim (15, 15') present on the respective rear side, and wherein
the swash plate (14) is arranged between the two drive wheels (18, 18'), which have a common axis of rotation (A), and the bevel gear rims (15, 15') are arranged facing each other on the axis of rotation (A).

2. The steering gear (13) according to claim 1,
**characterised in that**
the first traction means (16c) and the second traction means (16c') are closed or non-closed belts or ropes.

3. The steering gear (13) according to claim 1 or 2,
**characterised in that**
a drive axis (C) of the first motor (17) is parallel to a drive axis (C') of the second motor (17').

4. The steering gear (13) according to at least one of claims 1 to 3,
**characterised in that**
the spindle (16a, 16a') has a circumferential groove (16b, 16b') in which the traction means (16c, 16c') is guided.

5. The steering gear (13) according to at least one of claims 1 to 4,
**characterised in that**
the spindle (16a, 16a') has a spindle nut (16d, 16d').

6. The steering gear (13) according to at least one of claims 1 to 5,
**characterised in that**
the traction pulley (19, 19') has a circumferential groove (19a, 19a') in which the traction means (19c, 19c') rests, and/or
the traction pulley (19, 19') has a circumferential groove (19a, 19a') in which the ends of the non-closed traction means (19c, 19c') are fastened with sliding blocks.

7. The steering gear (13) according to at least one of claims 1 to 6,
**characterised in that**
each of the motors (17, 17') can be arranged via its respective spindle (16a, 16a') in any position pointing radially away from the respective drive wheel rim (19, 19'), wherein, in a preferred arrangement, the two drive axes (C, C') run parallel to each other.

8. The steering gear (13) according to at least one of claims 1 to 7,
**characterised in that**
the swash plate (14) is coupled to a third gear wheel (25) which is in engagement with the two bevel gear rims (15, 15') of the two double wheels (18, 18') and whose axis of rotation (D) is at a right angle to the common axis (A) of the driven double wheels (18, 18'), and/or
the swash plate (14) is coupled to a fourth gear wheel (31) which is coupled to the two bevel gear rims (15, 15') of the two double wheels (18, 18') and is arranged on the side facing away from the third gear wheel (25).

9. A surgical instrument (1) comprising a shaft (2), an actuation unit (4) arranged at the proximal end (3) of the shaft (2) and an instrument (7) arranged at the distal end (5) of the shaft (2) with a tool tip (6) which can be angled by means of a distal bending mechanism (9) and which can be controlled by a swash plate (14) which can be spatially aligned by means of two drives,
**characterised in that**
the surgical instrument (1) has a steering gear (13) according to at least one of claims 1 to 8, which has the two drives and is designed to transmit the adjustment angles of the two drives to the spatial orientation of the swash plate (14).

10. The surgical instrument (1) according to claim 9,
**characterised in that**
the swash plate (14), for coupling with a third gear wheel (25) which is in engagement with the two bevel gear rims (15, 15') of the two double wheels (18, 18'), is mounted rotatably about the longitudinal axis (B) of the shaft (2) via a bearing ring (32) in a steering ring (30), which is coupled to the third gear wheel (25) in a rotationally-fixed manner, wherein the swash plate (14) is coupled in a cardanic manner to a main shaft (21) running coaxially to a longitudinal axis (B) of the shaft (2).

11. The surgical instrument (1) according to claim 9 or 10,
**characterised in that**
the swash plate (14) is pivotably mounted on a universal joint disc (28) via two bearing pins (27) arranged offset by 180° relative to one another, wherein the universal joint disc (28) is pivotably mounted on the main shaft (21) via two bearing pins (29) arranged offset by 180° relative to one another and wherein the bearing pins (27, 29) of the swash plate (14) and the universal joint disc (28) are arranged offset by 90° relative to one another,
or
the cardanic mounting is provided by two longitudinally extending guide grooves (20a) located diametrically in the main shaft (21) and two pins (29) arranged diametrically and pointing radially inwards on the swash plate (14), wherein each pin (29) engages into one of the guide grooves (20a) such that an angle of rotation of the main shaft (21) can be transmitted to the swash plate (14).

12. The surgical instrument (1) according to at least one of claims 9 to 11, the fourth gear wheel (31) is coupled to the swash plate (14) via a bearing ring (42) with the steering ring (30), wherein the fourth gear wheel (31) is freely rotatable relative to the third gear wheel (25).

13. The surgical instrument (1) according to at least one of claims 9 to 12, **characterised in that**
an actuating element (8) is mounted so as to be axially displaceable in the shaft (2) and is operatively connected to the actuation unit (4) proximally at the side, and **in that** the distal bending mechanism (9) of the bendable tool tip (6) comprises swivel members (11) which are arranged at the distal end (5) of the shaft (2) and which are connected to the steering gear (13) via steering wires (12) extending in the longitudinal direction of the shaft (2).

14. The surgical instrument (1) according to at least one of claims 9 to 13, **characterised in that**
a radial distance of the steering wires (12) from the longitudinal axis (B) of the shaft (2) is greater at the swash plate (14) than at the proximal end (3) of the shaft (2) from which the steering wires (12) emerge, wherein
- the steering wires (12) extend from the proximal end (3) of the shaft (2) directly to the swash plate (14), wherein the steering wires (12) form an angle of less than 90° in relation to a disc surface of the swash plate (14), or
- on the distal side in front of the swash plate (14) is arranged a serrated lock washer (22) on the main shaft (21), which increases the radial distance of the steering wires (12) from the longitudinal axis (B) of the shaft (2) such that the steering wires (12) run parallel to each other between the serrated lock washer (22) and the swash plate (14) and form an angle of 90° in relation to a disc surface of the swash plate (14).

## Revendications

1. Appareil de direction (13) pour un instrument chirurgical (1) disposé à l'extrémité proximale (3) d'un arbre (2), qui définit un axe longitudinal (B) et a un mécanisme de flexion (9) au niveau de l'extrémité distale (5),
dans lequel l'appareil de direction (13) a deux entraînements motorisés et est conçu pour aligner dans l'espace un plateau oscillant (14) par l'intermédiaire des angles de réglage des deux entraînements, ce plateau étant conçu de manière à commander un mécanisme de flexion distal (9) de l'instrument chirurgical (1), dans lequel
le premier entraînement a un premier arbre d'entraînement (17a) entraîné par un premier moteur (17) et le second entraînement a un second arbre d'entraînement (17a') entraîné par un second moteur (17'), **caractérisé en ce que** le premier arbre d'entraînement (17a) est relié de manière opérationnelle à une première poulie de traction (19) d'une première roue d'entraînement (18) par l'intermédiaire d'un premier moyen de traction (16c) disposé entre la première poulie de traction (19) et une première broche (16a) montée sur le premier arbre d'entraînement (17a), qui définit un premier axe d'entraînement (C), et
le second arbre d'entraînement (17a') est relié de manière opérationnelle à une seconde poulie de traction (19') d'une seconde roue d'entraînement (18') par l'intermédiaire d'un second moyen de traction (16c'), qui est disposée entre la seconde poulie de traction (19') et une seconde broche (16a'), qui repose sur le second arbre d'entraînement (17a'), qui définit un second axe d'entraînement (C'),
dans lequel les première et deuxième roues d'entraînement (18, 18') sont conçues comme des roues doubles (18, 18') et ont chacune la poulie de traction (19, 19') correspondante et une couronne conique (15, 15') présente sur le côté arrière respectif, et dans lequel
le plateau oscillant (14) est disposé entre les deux roues d'entraînement (18, 18'), qui ont un axe de rotation commun (A), et les couronnes coniques (15, 15') sont disposées face à face sur l'axe de rotation (A).

2. Appareil de direction (13) selon la revendication 1,
**caractérisé en ce que**
le premier moyen de traction (16c) et le second moyen de traction (16c') sont des courroies ou des cordes fermées ou non fermées.

3. Appareil de direction (13) selon la revendication 1 ou 2,
**caractérisé en ce que**
un axe d'entraînement (C) du premier moteur (17) est parallèle à un axe d'entraînement (C') du second moteur (17').

4. Appareil de direction (13) selon au moins l'une des revendications 1 à 3, **caractérisé en ce que**
la broche (16a, 16a') a une rainure circonférentielle (16b, 16b') dans laquelle le moyen de traction (16c, 16c') est guidé.

5. Appareil de direction (13) selon au moins l'une des revendications 1 à 4, **caractérisé en ce que**
la broche (16a, 16a') a un écrou de broche (16d, 16d').

6. Appareil de direction (13) selon au moins l'une des revendications 1 à 5, **caractérisé en ce que**
la poulie de traction (19, 19') a une rainure circonférentielle (19a, 19a') dans laquelle le moyen de traction (19c, 19c') repose, et/ou
la poulie de traction (19, 19') a une rainure circonférentielle (19a, 19a') où les extrémités du moyens de traction non fermé (19c, 19c') sont fixés à l'aide de blocs coulissants.

7. Appareil de direction (13) selon au moins l'une des revendications 1 à 6, **caractérisé en ce que**
chacun des moteurs (17, 17') peut être disposé par l'intermédiaire de sa broche (16a, 16a') respective dans l'une quelconque position s'éloignant radialement de la jante de la roue d'entraînement (19, 19'), dans lequel, dans un arrangement préférentiel, les deux axes d'entraînement (C, C') sont parallèles l'un à l'autre.

8. Appareil de direction (13) selon au moins l'une des revendications 1 à 7, **caractérisé en ce que**
le plateau oscillant (14) est couplé à une troisième roue dentée (25) qui est en prise avec les deux couronnes coniques (15, 15') des deux roues doubles (18, 18') et dont l'axe de rotation (D) est à angle droit par rapport à l'axe commun (A) des roues doubles entraînées (18, 18'), et/ou
le plateau oscillant (14) est couplé à une quatrième roue dentée (31) qui est couplée aux deux couronnes coniques (15, 15') des deux roues doubles (18, 18') et qui est disposée du côté opposé à la troisième roue dentée (25).

9. Instrument chirurgical (1) comprenant un arbre (2), une unité d'actionnement (4) disposée à l'extrémité proximale (3) de l'arbre (2) et un instrument (7) disposé à l'extrémité distale (5) de l'arbre (2) avec une pointe d'outil (6) qui peut être inclinée au moyen d'un mécanisme de flexion distale (9) et qui peut être contrôlée par un plateau oscillant (14) qui peut être aligné dans l'espace au moyen de deux entraînements,
**caractérisé en ce que**
l'instrument chirurgical (1) a un appareil de direction (13) selon au moins l'une des revendications 1 à 8, qui a les deux entraînements et qui est conçu pour transmettre les angles de réglage des deux entraînements à l'orientation spatiale du plateau oscillant (14).

10. Instrument chirurgical (1) selon la revendication 9,
**caractérisé en ce que**
le plateau oscillant (14), pour l'accouplement avec une troisième roue dentée (25) qui est en prise avec les deux couronnes coniques (15, 15') des deux roues doubles (18, 18'), est monté rotatif autour de l'axe longitudinal (B) de l'arbre (2) par l'intermédiaire d'une bague de roulement (32) dans une bague de direction (30), qui est couplée à la troisième roue dentée (25) de manière fixe en rotation, dans lequel le plateau oscillant (14) est couplé de manière cardanique à un arbre principal (21) coaxial à l'axe longitudinal (B) de l'arbre (2).

11. Instrument chirurgical (1) selon la revendication 9 ou 10,
**caractérisé en ce que**
le plateau oscillant (14) est monté pivotant sur un disque de joint universel (28) par l'intermédiaire de deux goupilles d'appui (27) décalés de 180° l'un par rapport à l'autre, dans lequel le disque de joint universel (28) est monté pivotant sur l'arbre principal (21) par l'intermédiaire de deux goupilles d'appui (29) décalés de 180° l'un par rapport à l'autre et dans lequel les goupilles d'appui (27, 29) du plateau oscillant (14) et du disque de joint universel (28) sont décalés de 90° l'un par rapport à l'autre,
ou
le montage cardanique est fourni par deux rainures de guidage (20a) s'étendant longitudinalement et situées diamétralement dans l'arbre principal (21) et deux goupilles (29) disposées diamétralement et orientées radialement vers l'intérieur sur le plateau oscillant (14), dans lequel chaque goupille (29) s'engage dans l'une des rainures de guidage (20a) de sorte qu'un angle de rotation de l'arbre principal (21) puisse être transmis au plateau oscillant (14).

12. Instrument chirurgical (1) selon au moins l'une des revendications 9 à 11, la quatrième roue dentée (31) est couplée au plateau oscillant (14) par l'intermédiaire d'une bague de roulement (42) avec la bague de direction (30), dans lequel la quatrième roue dentée (31) peut tourner librement par rapport à la troisième roue dentée (25).

13. Instrument chirurgical (1) selon au moins l'une des revendications 9 à 12, **caractérisé en ce que**
un élément d'actionnement (8) est monté de manière à pouvoir être déplacé axialement dans l'arbre (2) et est relié de manière opérationnelle à l'unité d'actionnement (4) proximalement sur le côté, et **en ce que** le mécanisme de flexion distale (9) de la pointe de l'outil pliable (6) comprend des éléments pivotants (11) qui sont disposés à l'extrémité distale (5) de l'arbre (2) et qui sont reliés à l'appareil de direction (13) par des fils de direction (12) s'étendant dans la direction longitudinale de l'arbre (2).

14. Instrument chirurgical (1) selon au moins l'une des revendications 9 à 13, **caractérisé en ce que**
une distance radiale des fils de direction (12) depuis l'axe longitudinal (B) de l'arbre (2) est plus grande au niveau du plateau oscillant (14) qu'à l'extrémité proximale (3) de l'arbre (2) d'où sortent les fils de direction (12), dans lequel
- les fils de direction (12) s'étendent de l'extrémité proximale (3) de l'arbre (2) directement au plateau oscillant (14), dans lequel les fils de direction (12) forment un angle inférieur à 90° par rapport à la surface du disque du plateau oscillant (14), ou
- sur le côté distal, devant le plateau oscillant (14), une rondelle éventail (22) est disposée sur l'arbre principal (21), qui augmente la distance radiale des fils de direction (12) depuis l'axe longitudinal (B) de l'arbre (2) de telle sorte que les fils de direction (12) soient parallèles entre la rondelle éventail (22) et le plateau oscillant (14) et forment un angle de 90° par rapport à la surface du disque du plateau oscillant (14).
